# EUROPEAN PATENT SPECIFICATION

(11) **EP 2 967 752 B1**
(45) Date of publication and mention of the grant of the patent: **07.03.2018**
(21) Application number: 14779617.1
(22) Date of filing: 11.03.2014
(51) Int. Cl.: A61B 18/20, A61B 18/00

(54) **FRACTIONAL HANDPIECE FOR DERMATOLOGICAL TREATMENTS**
FRAKTIONIERTES HANDSTÜCK FÜR DERMATOLOGISCHE BEHANDLUNGEN
PIÈCE MANUELLE FRACTIONNELLE POUR TRAITEMENTS DERMATOLOGIQUES

(30) Priority: 11.03.2013 US 201361776327 P
(43) Date of publication of application: 20.01.2016
(73) Proprietor: Biolase, Inc., Irvine, CA 92618 (US)
(72) Inventor: BOUTOUSSOV, Dmitri, Dana Point, CA 92629 (US); NETCHITAILO, Vladimir, Livermore, CA 94551 (US); SALEHI, Maziar, Irvine, CA 92604 (US); CELY, Julio, Mission Viejo, CA 92691 (US); LEMBERG, Vladimir, Santa Clara, CA 95054 (US)
(74) Representative: Wachenhausen & Kollegen Patentanwälte GbR
(86) International application number: PCT/US2014/023365
(87) International publication number: WO 2014/164737

(56) References cited:
- WO-A1-2008/124839
- WO-A1-2012/090180
- WO-A1-2012/090180
- DE-A1- 3 527 451
- US-A1- 2005 143 719
- US-A1- 2006 149 343
- US-A1- 2007 260 230
- US-A1- 2008 058 783
- US-A1- 2012 253 334
- US-A1- 2012 283 712
- US-B1- 6 251 102
- US-B1- 6 544 256

## Description

### TECHNICAL FIELD

This relates to a dermatological laser treatment device used for fractional laser treatment of skin.

### BACKGROUND

A fractional laser treatment device is used to treat skin conditions. A medical practitioner holds the device against a patient's skin. The device emits, toward the skin, laser pulses that produce a matrix of small ablation areas on the skin. The ablation areas are later filled in by growth from surrounding unablated skin tissue, which results in improved skin texture.

WO 2012/090180 A1 relates to a device for fractional laser therapy to affect dermatological treatment, comprising a trigger functionally associated with a distance measurer, which is functionally associated with a contact surface, the trigger being functionally associated with a light source.

### SUMMARY

A medical device includes a housing that is moved along a surface of a target tissue in a longitudinal direction. One or more supply lines conduct air and water to the housing. A pulse emitter emits electromagnetic pulses toward the surface at a repetition rate for the pulses to produce ablation holes in the tissue. The pulse emitter includes optical components and is configured to direct the air against the optical components to keep the optical components clean. One or more nozzles emit the water and the air in an air/water spray to moisturize and cool the target tissue prior to laser application.

A medical device includes a housing configured to be moved along a surface of a target tissue in a longitudinal direction. A movement sensor measures a movement parameter, which might be longitudinal speed or displacement, of the housing relative to the surface. A pulse emitter emits electromagnetic pulses toward the surface at a repetition rate, for the pulses to produce ablation holes in the tissue. A controller controls the repetition rate, based on the measured movement parameter, so that the ablation holes are spaced apart along the longitudinal direction.

In one example, the pulses are laser pulses. The movement parameter is longitudinal speed, the sensor outputs a speed signal indicative of the longitudinal speed, and the controller controls the repetition rate to be a function of the longitudinal speed indicated by the speed signal. Alternatively, the movement parameter is longitudinal displacement, the sensor is configured to output a displacement signal indicative of the longitudinal displacement, and the controller is configured to control emission of the pulses to be a function of the displacement signal.

In one example, the sensor includes a roller that is rotatably secured to the housing and configured to roll against the surface as the housing is moved longitudinally along the surface, so that rate of rotation of the roller is proportional to speed of the longitudinal movement. The sensor is configured measure an angular movement parameter, which may be angular speed or angular displacement, of the roller. The angular movement parameter may be angular speed of the roller, and the controller may control the repetition rate to be a function of the angular speed. Alternatively, the angular movement parameter is angular displacement of the of the roller, and the controller controls the laser emitter to space apart the pulses as a function of the angular displacement.

In one example, the roller is a first roller at a first side of the housing and the angular movement parameter is a first angular movement parameter. The movement sensor includes a second roller at a laterally opposite second side of the housing, that is rotatably secured to the housing to rotate independently of rotation of the first roller and to roll against the surface. The movement sensor measures a second angular movement parameter, comprising angular speed or angular displacement, of the second roller.

### BRIEF DESCRIPTION OF THE FIGURES

FIG. 1 is a schematic diagram of an example medical laser system, including a laser handpiece, for performing fractional laser treatment procedures.

FIG. 2 is a perspective view of the handpiece and ablation holes generated by the handpiece.

### DETAILED DESCRIPTION

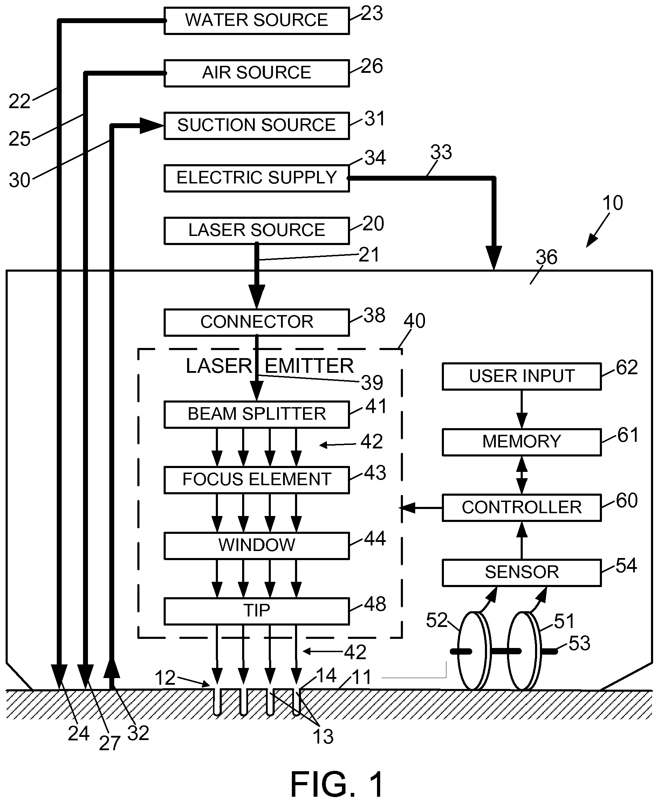

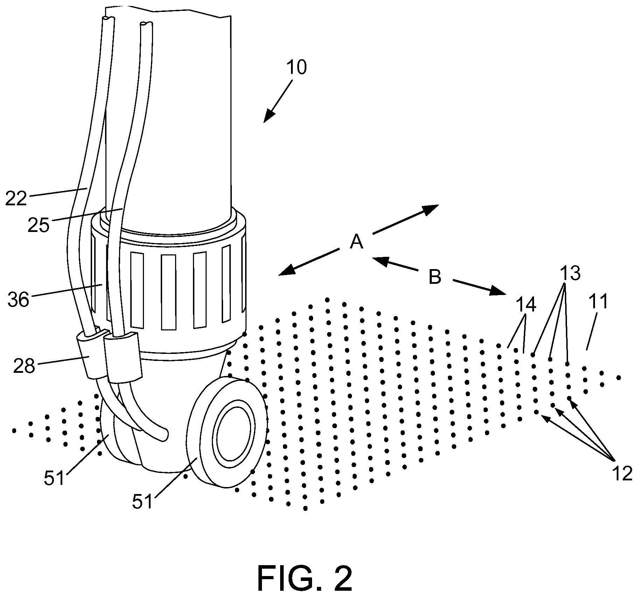

Fig. 1 is a block diagram of a medical system for electromagnetic beam treatment of biological tissue. The system includes a handpiece 10, a perspective view of which is shown in Fig. 2. Referring to Figs. 1-2, a medical practitioner (user) holds the handpiece 10 against a surface 11 of the target tissue to be treated. As the user moves the handpiece 10 along the surface 11, the handpiece 10 emits electromagnetic pulses (short-duration electromagnetic energy beams) toward the tissue surface. The tissue is ablated in a fractional manner, in that the pulses are timed and oriented so as to ablate an array of small areas 13 in the tissue. The handpiece 10 tracks its progress in traversing the surface 11 in order to time the pulses to be properly spaced apart along the surface 11. Unablated tissue 14 surrounding each ablated area will then quickly rejuvenate the small ablated areas 13.

In this example, the target tissue is skin, the treatment is a dermatological treatment, the electromagnetic pulse is a laser pulse, and the handpiece is a fractional laser handpiece. The ablated area 13 may be termed as an ablation, perforation, spot, pixel, dot, hole, pit or crater. Each perforation 13 stimulates growth of new collagen. The new collagen improves the skin's texture and tone to smooth fine lines and wrinkles. Unablated skin 14 surrounding each perforation remains intact, which allows the skin's top layer 11 to heal rapidly from the edge 14 of each perforation 13.

In the following description of the system's hardware, the handpiece 10 is configured to be moved along the surface 11 in a "longitudinal" direction (arrow "A" in Fig. 2), which is perpendicular to a "lateral" direction along the surface 11 (arrow "B" in Fig. 2).

The example system includes an ablation energy source 20 that generates the electromagnetic energy for ablating the tissue 11. In this example, the source 20 is a laser source that generates laser energy. Example laser sources are a Er,Cr:YSGG laser, a Er:YAG laser, a diode laser, a Nd:YAG laser, an Argon laser module or C02 laser module. Wavelength in this example is in the range of 1.9-3.0 micrometers, and may be in the rage 2.7-2. 85 micrometers range, as this provides a deeper penetration than Er:YAG. Energy density within each spot should be above the ablation threshold of the tissue being treated. The energy density to be used for each type of treatment may be determined based on a balance of pulse energy and number of spots and spot size.

In this example, the laser source 20 is outside the handpiece 10. It may alternatively be within, and part of, the handpiece 10.

An ablation energy supply line 21, which may include one or more optical fibers, conducts the electromagnetic energy, in this example laser energy, from the ablation energy source 20 to the handpiece 10. A water supply line 22 conducts water from a water source 23 to a water nozzle 24 at the front (laser emitting end) of the handpiece 10. An air supply line 25 conducts air from an air source 26 (air pump) to an air nozzle 27 at the front of the handpiece 10. A suction line 30 provides suction from a suction source 31 to a suction port 32 at the front of the handpiece 10. An electrical supply line 33 provides electrical power from a power supply 34 to circuitry (e.g., controller) of the handpiece 10.

The nozzles 24, 27 emit and direct the water and air to the skin surface 11. The nozzles emit the water and the air in the form of an air/water spray that moisturizes and cools the tissue prior to laser application. The air and water, along with the suction, keep the treatment area 11 cool and free from debris. The water may be sprayed on the treatment area 11 in a fine high-pressure mist to cool and wet the treatment area, react with the laser pulse to achieve the ablation, and stimulate ablation of superficial less-hydrated skin. The air and the suction may also be channeled into the handpiece 10 to keep optical components of the handpiece 10 clean.

The handpiece 10 includes a housing 36 configured to be gripped by the user and moved along the skin surface 11. The housing 36 is ergonomically designed to facilitate holding and operating of the handpiece 10.

A connector 38 at a rear end of the housing 36 is removably coupled to the laser energy supply line 21 to receive the laser energy. Optical fibers 39 within the housing 36 channel the laser energy from the connector 38 to a laser pulse emitter 40 within the housing 36.

The pulse emitter 40 in this example includes the following components: A beam splitter 41, such as an optical assembly with a diffractive optical element, splits the laser energy 39 into a laterally extending row (series) of separate laser pulses 42 that are laterally spaced apart. The laser pulses are emitted toward the surface 11 so as to produce a laterally extending row 12 of ablation holes. The laser pulses for each row 42 may be emitted simultaneously or sequentially. The lateral spacing distance between holes is user selectable. A focusing element 43 includes one or more lens elements for focusing the laser pulses. A protective window 44 protects the optical components from damage or debris. A laser tip 48 is secured to the end of the emitter 40. The tip 48 may contact the skin surface 11 when applying the laser pulses to the skin. The tip 48 may be disposable, so that a different sanitary tip may be used for each patient. The emitter 40 includes optical components and is configured to direct the air against the optical components to keep the optical components clean.

Support rollers 51, 52 (wheels) of the handpiece 10 are rotatably secured to the housing 36 by an axle 53 at laterally opposite sides of the housing 36. The rollers 51, 52 are configured to roll against the surface 11 as the housing 36 is moved longitudinally along the surface 11. When the handpiece 10 is pressed against and moved along the surface 11, the rollers 51, 52 eliminate shear forces and scraping of the surface, which might occur in the absence of rollers. The rollers 51, 52 also keep the laser optics optimally, precisely and consistently positioned above the skin for optimal and uniform focusing of the laser light as the handpiece 10 is moved along the surface 11. In this example, the handpiece 10 has two rollers 51, 52 at laterally opposite sides of the handpiece 10 to keep both sides of the handpiece 10 uniformly spaced from the surface 11, which reduces lateral tilting of the handpiece 10. A third roller (not shown), longitudinally offset from the other two rollers 51, 52, may be added to avoid longitudinal tilting of the handpiece 10. The rollers 51, 52 in this example are made of plastic, and are transparent to avoid blocking the user's view of the treatment area.

A movement sensor 54 in this example senses (e.g., measures) a linear movement parameter, which comprises longitudinal speed or longitudinal displacement (where "or" includes the possibility of both), of the housing 36 relative to the surface 11. The sensor 54 senses the handpiece's longitudinal movement indirectly, by measuring movement of a movable element that moves synchronously (in sync) with the handpiece's longitudinal movement. In this example, the moveable element comprises one of the support rollers -- roller 51 -- that serves as a tracking roller (tracking wheel). A rate of rotation of the tracking roller 51 is proportional to speed of the handpiece's longitudinal movement. The sensor 54 senses an angular movement parameter, comprising the roller's angular speed or angular displacement, and outputs a signal that indicates an angular movement parameter (angular speed or displacement) which is also indicative of the linear movement parameter (longitudinal speed or displacement). The sensing may be based on a Hall effect sensor that detects a magnet attached to the tracking roller 51. Or the sensing may be based on an optical reader that detects interruptions in a light beam that is transmitted through a grating in the tracking roller 51 (as do some mouse mechanisms), or interruptions in a light beam that is reflected off of reflectors that are circumferentially spaced apart about the tracking roller 51. Or the sensing may be based on a mechanical electrical switch whose activation lever is pressed by protrusions that are circumferentially spaced apart about the tracking roller 51. In these examples, having at least two magnets or at least two light beams or at least two switches enables the sensor 54 to also sense direction (i.e., in addition to speed and displacement).

In one example, the rollers 51, 52 are coupled together with a common axle 53 that forces the rollers 51, 52 to rotation in unison. In that case, the sensing device 54 might monitor movement of only one of the rollers 51.

In another example, the rollers 51, 52 rotate independently of each other. In that case, the sensor 54 may sense the angular movement parameter (i.e., angular speed or displacement) of each roller 51, 52 independently, to yield two independent angular movement parameters. The sensor 54 may then sense (measure), based on the imbalance in rotation of the two rollers 51, 52, the curvature of the path of movement along the surface 11. This is especially useful where the handpiece 10 is moved along the surface 11 in an arcuate (curved) path of small radius, such that the roller at the outside of the arc moves significantly faster than the roller at the inside of the arc.

A controller 60 in the housing 36 receives the output signal of the movement sensor 54 and uses the output signal to time the pulses to be longitudinally spaced apart in a controlled manner. The longitudinal hole spacing (longitudinal distance between holes) can be reproducible and uniform despite variability in the handpiece's speed. That is because the controller 60 controls the repetition rate of the pulses 42, and thus the longitudinal spacing between holes 13, to be a function of the sensed angular movement parameter (angular speed or displacement), which is in turn a function of the linear movement parameter (longitudinal speed or displacement). For example, the controller 60 may space apart the pulses as a function of the angular displacement. The repetition rate might be proportional to the longitudinal speed if the longitudinal spacing is to be uniform. The controller 60 may cause the pulses to cease when the handpiece 10 stops moving, and automatically restart when the handpiece 10 starts moving again.

In an example that does not employ a movement sensor to adjust the repetition rate to correspond to longitudinal speed, the user moves of the handpiece 10, while the pulses are being emitted, at a speed that corresponds to pulse rate and longitudinal hole spacing in a single exposure to produce a uniform pattern. Even in such a scenario where a movement sensor is not used, the holes in each laterally-extending row may be produced simultaneously or sequentially (in a scanned manner).

If the sensor 54 senses direction (as described above), the controller 60 may detect when roller rotation has reversed direction, which indicates the handpiece 10 has reversed longitudinal direction and is returning to an area that was already ablated. In that scenario, the controller 60 would control the pulse emitter 40 to cease emitting pulses, which is equivalent to dropping the repetition rate to zero, so as not to ablate the same area twice.

If the sensor 54 sense curvature (such as by using the two-roller configuration described above), the controller 60 may control the repetition rate as a function of path curvature. For example, the controller 60 may detect that one side of the handpiece 10 is moving slower than the laterally opposite side of the handpiece 10. In such a scenario, the controller 60 might control the emitter 40 for the repetition rate to increase near the outside of the arc and/or decrease near the inside of the arc, so as to maintain a uniform hole density (i.e., keep the hole density the same at the inside of the arc as at the outside of the arc). Alternatively, or in addition, the controller 60 might control the emitter 40 for the lateral hole spacing to increase along the inside of the curve and decrease along the outside of the curve. The controller 60 might also use the curvature sensing to keep track of the handpiece's path. That would enable the controller 60 to detect when the handpiece's path of movement has curved around and is crossing over itself. The controller 60 might respond by ceasing laser pulses (i.e., lowering the repetition rate to zero) so as not to ablate the same area twice.

The controller 60 may be a mechanical device, or based on hardwired electronic logic such with an ASIC (application specific integrated circuit), or based on a microprocessor that executes program instructions stored in a memory 61 of the handpiece 10 to perform its functions. Besides storing program code, the memory 61 might be used to store sensed information regarding prior use of the handpiece, such as data that indicates the path that has been covered by the handpiece 10, to avoid ablating the same area twice.

In the procedure described above, the laterally extending rows 12 of holes 13 are spaced longitudinally apart. This results in an array (matrix) of holes 13 that are arranged in laterally extending rows 12 and longitudinally extending columns. The repetition rate of the laser pulses, and thus the row spacing (spacing between rows), is a function of the angular movement parameter (angular speed or displacement) of at least one of the rollers 51, which is in turn a function of the linear movement parameter (longitudinal speed or displacement).

During a single stroke of the handpiece 10 along the surface 11, the handpiece 10 may generate from two holes to an array of tens of thousands of holes or more. The array is a one-dimensional or two-dimensional array of ablation holes. By limiting the ablation to an array of small holes 13, the handpiece 10 might treat only 15-20 percent of the treatment area. The laser pulses 42 create, at each hole 13, an ablative thermal channel, creating a micro-injury, without disturbing the surrounding tissue. The micro-injured areas start the healing process and the surrounding untreated area 14 acts as a reservoir for rapid restoration. As collagen remodels, skin tightens, and texture and scars improve. The intact, undamaged skin around the treatment site promotes quicker healing for faster recovery.

The handpiece 10 has a user input device 62, such as a keypad or application-specific buttons or touch screen, that enables the user to set treatment parameters. The user selectable parameters may include number of holes per row, lateral spacing and longitudinal spacing between holes, hole size, and laser power intensity and pulse duration which affect ablation depth. The user may specify whether the column spacing and/or row spacing should be uniform and nonuniform, and (if nonuniform) specify what nonuniform spacing pattern to use. For example, the user may enter a selection in the input device 62 for the longitudinal spacing between holes to be non-uniform (changing), while keeping the lateral spacing within each row uniform and, if desired, keeping the lateral spacing within each row constant even from row to row.

Examples of treatment parameters are as follows: The number of pulsed beams, and thus the holes 13, in each row might be in the range 1-20, or more. The laser repetition rate might vary from 10 Hz to 15 Hz as handpiece 10 speed varies from 3.3 mm/sec to 10 mm/sec. The hole size might be in the range 150-250 um. The lateral spacing and longitudinal spacing between holes might be in the range 500-1000 um. The lateral spacing might be equal to, or not equal to, the longitudinal spacing. The laser energy in each hole may be in the range 5-20 mJ. In one example, the array of pixels may include ten columns with a column spacing (spacing between columns) in the range 600-1000 um. The handpiece's longitudinal speed along the surface 11 might be 66 mm/sec. An example hole diameter is 200 um. An example hole depth is 1 mm. There is no limit to the number of rows 12 of holes 13, since they are continually generated as the handpiece 10 is moving.

The fractional handpiece 10 described above is well suited for dermatological treatments of the face, chest, neck and hands. It is particularly well suited for reducing mild to medium wrinkling. It enables quick treatment time, with no requirement for anesthesia, gels or other disposables, and with little or no downtime, and reduces risk of complications.

The components and procedures described above provide examples of elements recited in the claims. They also provide examples of how a person of ordinary skill in the art can make and use the claimed invention. They are described here to provide enablement and best mode without imposing limitations that are not recited in the claims. In some instances in the above description, a term is followed by a substantially equivalent term enclosed in parentheses.

## Claims

1. A medical device (10) comprising:
a housing (36) configured to be moved along a surface (11) of a target tissue in a longitudinal direction (A); one or more supply lines (22, 25) configured to conduct air and water to the housing (36);
a pulse emitter (40) configured to emit electromagnetic pulses toward the surface (11) at a repetition rate for the pulses to produce ablation holes (13) in the tissue, wherein the pulse emitter (40) includes optical components (41, 43, 44) and is configured to direct the air against the optical components (41, 43, 44) to keep the optical components (41, 43, 44) clean;
**characterized in that** the medical device (10) further comprises
one or more nozzles (24, 27) configured to emit the air and the water in an air/water spray to moisturize and cool the target tissue prior to laser application.

2. The medical device (1 0) of claim 1, wherein the pulses generate laterally-extending rows (12) of the holes, and wherein the medical device (10) further includes:
a user input device (62) configured for a user to enter a selection for longitudinal spacing between the holes to be non-uniform while keeping lateral spacing within each row uniform.

3. The medical device (10) of claim 1, further comprising:
a movement sensor (54) configured to measure a movement parameter, comprising longitudinal speed or longitudinal displacement, of the housing (3 6) relative to the surface (11); and
a controller (60) configured to control the repetition rate, based on the measured movement parameter, so that the ablation holes are spaced apart along the longitudinal direction.

4. The medical device (10) of claim 1, wherein the electromagnetic pulses are laser pulses having a wavelength in the range of 1.9-3.0 micrometers.

5. The medical device (10) of claim 3, wherein the movement parameter comprises longitudinal speed, and the sensor (54) is configured to output a speed signal indicative of the longitudinal speed, and wherein the controller (60) is configured to control the repetition rate to be a function of the longitudinal speed indicated by the speed signal, or wherein the movement parameter comprises longitudinal displacement, and the sensor (54) is configured to output a displacement signal indicative of the longitudinal displacement, and wherein the controller (60) is configured to control emission of the pulses to be a function of the displacement signal.

6. The medical device (10) of claim 3, further comprising a roller (51; 52) that is rotatably secured to the housing (36) and configured to roll against the surface (11) as the housing (36) is moved longitudinally along the surface (11), so that rate of rotation of the roller (51; 52) is proportional to longitudinal speed, and wherein the movement sensor (54) is configured to measure an angular movement parameter, comprising angular speed or angular displacement, of the roller (51; 52).

7. The medical device (10) of claim 6, wherein the angular movement parameter is angular speed of the roller (51; 52), and the controller (60) is configured to control the repetition rate to be a function of the angular speed, or wherein the angular movement parameter is angular displacement of the roller (51; 51), and the controller (60) is configured to control the emitter (40) to space apart the pulses as a function of the angular displacement.

8. The medical device (10) of claim 6, wherein the roller is a first roller (51) at a first side of the housing (36), the angular movement parameter is a first angular movement parameter, the movement sensor (54) includes a second roller (52) at a laterally opposite second side of the housing (36), the second roller (52) is rotatably secured to the housing (36) to rotate independently of rotation of the first roller (51), and the second roller (52) is configured to roll against the surface (11), and the movement sensor (54) is configured to measure a second angular movement parameter, comprising angular speed or angular displacement, of the second roller (52).

9. The medical device (10) of claim 8, wherein the controller (60) is configured to control the repetition rate as a function of both the first movement parameter and the second angular movement parameter.

10. The medical device (10) of claim 8, wherein the pulses emitted by the emitter (40) generate laterally-extending (12) rows of ablation holes, and wherein the controller (60) is configured to control the number of ablation holes in each row as a function of both the first angular movement parameter and the second angular movement parameter.

11. The medical device (10) of claim 6, wherein the controller (60) is configured to drop the repetition rate to zero in response to the roller reversing direction.

12. The medical device (10) of claim 6, wherein the roller (51; 52) is transparent.

13. The medical device (10) of claim 3, wherein the pulses include pulses that are laterally spaced apart to produce laterally-extending rows (12) of ablation holes that, when combined with the longitudinal movement of the housing, yield a matrix of the holes (13) aligned in laterally extending rows (12) and longitudinally extending columns.

14. The medical device (10) of claim 13, wherein the pulse emitter (40) is configured to generate the holes in each row simultaneously or sequentially.

15. The medical device (10) of claim 13, wherein the emitter (40) includes a diffractive optical element (41) configured to split electromagnetic energy into a laterally extending row of simultaneous electromagnetic pulses that generate the holes simultaneously.

16. The medical device (10) of claim 13, wherein the controller (60) is configured for the ablation holes to be uniformly spaced apart along the longitudinal direction or to be non-uniformly spaced apart along the longitudinal direction.

17. The medical device (10) of claim 13, wherein the controller (60) is configured for the ablation holes to be uniformly spaced apart along the lateral direction or to be non-uniformly spaced apart along the lateral direction.

## Patentansprüche

1. Medizinische Vorrichtung (10), aufweisend :
ein Gehäuse (36), welches eingerichtet ist, um entlang einer Oberfläche (11) eines Zielgewebes in einer Längsrichtung (A) bewegt zu werden; eine oder mehrere Versorgungsleitungen (22, 25), die eingerichtet sind, um dem Gehäuse (36) Luft und Wasser zuzuführen;
einen Pulsemitter (40), der eingerichtet ist, um elektromagnetische Pulse in Richtung der Oberfläche (11) mit einer Pulswiederholrate auszusenden, um Ablationslöcher (13) in dem Gewebe zu erzeugen, wobei der Pulsemitter (40) optische Komponenten (41, 43, 44) aufweist und eingerichtet ist, um die Luft gegen die optischen Komponenten (41, 43, 44) zu leiten, um die optischen Komponenten (41, 43, 44) sauber zu halten;
**dadurch gekennzeichnet, dass** die medizinische Vorrichtung (10) ferner aufweist
eine oder mehrere Düsen (24, 27), die eingerichtet sind, um die Luft und das Wasser in einem Luft/Wasser-Spray auszugeben, um das Zielgewebe vor der Laserapplikation zu befeuchten und kühlen.

2. Medizinische Vorrichtung (10) nach Anspruch 1, bei der die Pulse sich quer erstreckende Reihen (12) der Löcher erzeugen und die medizinische Vorrichtung (10) ferner aufweist:
eine Benutzereingabevorrichtung (62), die so eingerichtet ist, dass ein Benutzer eine Auswahl für den Längsabstand zwischen den Löchern eingeben kann, die ungleichmäßig ist, wobei der Querabstand innerhalb jeder Reihe einheitlich gehalten wird.

3. Medizinische Vorrichtung (10) nach Anspruch 1, ferner aufweisend:
einen Bewegungssensor (54), der eingerichtet ist, um einen Bewegungsparameter zu messen, der Längsgeschwindigkeit oder Längsverschiebung des Gehäuses (36) relativ zur Oberfläche (11) umfasst; und
eine Steuerung (60), die eingerichtet ist, um die Wiederholrate basierend auf dem gemessenen Bewegungsparameter so zu steuern, dass die Ablationslöcher entlang der Längsrichtung voneinander entfernt sind.

4. Medizinische Vorrichtung (10) nach Anspruch 1, bei der die elektromagnetischen Pulse Laserimpulse mit einer Wellenlänge im Bereich von 1,9-3,0 Mikrometern sind.

5. Medizinische Vorrichtung (10) nach Anspruch 3, wobei der Bewegungsparameter die Längsgeschwindigkeit umfasst und der Sensor (54) so eingerichtet ist, dass er ein Geschwindigkeitssignal ausgeben kann, welches die Längsgeschwindigkeit anzeigt, und wobei die Steuerung (60) eingerichtet ist, um die Wiederholrate in Abhängigkeit der durch das Geschwindigkeitssignal angezeigten Längsgeschwindigkeit zu steuern, oder wobei der Bewegungsparameter die Längsverschiebung umfasst und der Sensor (54) so eingerichtet ist, dass er ein Verschiebungssignal ausgeben kann, welches die Längsverschiebung anzeigt, und wobei die Steuerung (60) eingerichtet ist, um die Ausgabe der Pulse in Abhängigkeit des Verschiebungssignals zu steuern.

6. Medizinische Vorrichtung (10) nach Anspruch 3, ferner mit einer Rolle (51; 52), die drehbar am Gehäuse (36) befestigt und so ausgebildet ist, dass sie entlang der Oberfläche (11) rollt, wenn das Gehäuse (36) längs entlang der Oberfläche (11) bewegt wird, sodass die Rotationsrate der Rolle (51; 52) proportional zur Längsgeschwindigkeit ist, wobei der Bewegungssensor (54) eingerichtet ist, um einen Winkelbewegungsparameter der Rolle (51; 52) zu messen, der Winkelgeschwindigkeit oder Winkelverschiebung umfasst.

7. Medizinische Vorrichtung (10) nach Anspruch 6, bei welcher der Winkelbewegungsparameter die Winkelgeschwindigkeit der Rolle (51; 52) ist und die Steuerung (60) eingerichtet ist, um die Wiederholrate in Abhängigkeit der Winkelgeschwindigkeit zu steuern, oder bei welcher der Winkelbewegungsparameter die Winkelverschiebung der Rolle (51; 52) ist und die Steuerung (60) eingerichtet ist, um den Emitter (40) so zu steuern, dass die Pulse in Abhängigkeit von der Winkelverschiebung voneinander entfernt werden.

8. Medizinische Vorrichtung (10) nach Anspruch 6, bei der die Rolle eine erste Rolle (51) an einer ersten Seite des Gehäuses (36) ist, der Winkelbewegungsparameter ein erster Winkelbewegungsparameter ist, der Bewegungssensor (54) eine zweite Rolle (52) an einer quer gegenüberliegenden zweiten Seite des Gehäuses (36) aufweist, die zweite Rolle (52) drehbar am Gehäuse (36) befestigt ist, um sich unabhängig von der Drehung der ersten Rolle (51) zu drehen, und die zweite Rolle (52) eingerichtet ist, um gegen die Oberfläche (11) zu rollen, und der Bewegungssensor (54) eingerichtet ist, um einen zweiten Winkelbewegungsparameter der zweiten Rolle (52) zu messen, der Winkelgeschwindigkeit oder Winkelverschiebung umfasst.

9. Medizinische Vorrichtung (10) nach Anspruch 8, bei der die Steuerung (60) eingerichtet ist, um die Wiederholrate in Abhängigkeit von sowohl dem ersten Bewegungsparameter als auch dem zweiten Winkelbewegungsparameter zu steuern.

10. Medizinische Vorrichtung (10) nach Anspruch 8, bei der die von dem Emitter (40) ausgegebenen Pulse sich quer erstreckende (12) Reihen von Ablationslöchern erzeugen, und bei der die Steuerung (60) eingerichtet ist, um die Anzahl von Ablationslöcher in jeder Reihe in Abhängigkeit von sowohl dem ersten Winkelbewegungsparameter als auch dem zweiten Winkelbewegungsparameter zu steuern.

11. Medizinische Vorrichtung (10) nach Anspruch 6, bei der die Steuerung (60) eingerichtet ist, um die Wiederholrate als Reaktion auf die Drehrichtungsumkehr der Rolle auf null abzusenken.

12. Medizinische Vorrichtung (10) nach Anspruch 6, bei der die Rolle (51; 52) transparent ist.

13. Medizinische Vorrichtung (10) nach Anspruch 3, bei der die Pulse quer voneinander beabstandete Pulse umfassen, um sich quer erstreckende Reihen (12) von Ablationslöchern zu erzeugen, die, wenn sie mit der Längsbewegung des Gehäuses kombiniert werden, zu einer Matrix der Löcher (13) führen, die in sich quer erstreckende Reihen (12) und sich längs erstreckende Spalten ausgerichtet ist.

14. Medizinische Vorrichtung (10) nach Anspruch 13, bei welcher der Pulsemitter (40) eingerichtet ist, um die Löcher in jeder Reihe gleichzeitig oder sequentiell zu erzeugen.

15. Medizinische Vorrichtung (10) nach Anspruch 13, bei welcher der Emitter (40) ein brechendes optisches Element (41) umfasst, das eingerichtet ist, um elektromagnetische Energie in eine sich quer erstreckende Reihe von simultanen elektromagnetischen Pulsen aufzuspalten, welche die Löcher gleichzeitig erzeugen.

16. Medizinische Vorrichtung (10) nach Anspruch 13, bei welcher die Steuerung (60) so eingerichtet ist, dass die Ablationslöcher entlang der Längsrichtung gleichmäßig voneinander entfernt sind oder entlang der Längsrichtung ungleichmäßig voneinander entfernt sind.

17. Medizinische Vorrichtung (10) nach Anspruch 13, bei welcher die Steuerung (60) so eingerichtet ist, dass die Ablationslöcher entlang der Querrichtung gleichmäßig voneinander entfernt sind oder entlang der Querrichtung ungleichmäßig voneinander entfernt sind.

## Revendications

1. Dispositif médical (10) comportant :
un boîtier (36) configuré pour être déplacé le long d'une surface (11) d'un tissu cible dans une direction longitudinale (A) ; une ou plusieurs canalisations (22, 25) d'alimentation configurées pour acheminer de l'air et de l'eau jusqu'au boîtier (36) ;
un émetteur (40) d'impulsions configuré pour émettre des impulsions électromagnétiques vers la surface (11) à un taux de répétition pour que les impulsions produisent des trous (13) d'ablation dans le tissu, l'émetteur (40) d'impulsions comprenant des composants optiques (41, 43, 44) et étant configuré pour diriger l'air contre les composants optiques (41, 43, 44) afin de maintenir les composants optiques (41, 43, 44) propres ;
**caractérisé en ce que** le dispositif médical (10) comporte en outre
une ou plusieurs buses (24, 27) configurées pour émettre l'air et l'eau en une pulvérisation air/eau afin d'humidifier et de refroidir le tissu cible avant l'application du laser.

2. Dispositif médical (10) selon la revendication 1, les impulsions générant des lignes (12) des trous s'étendant latéralement, et le dispositif médical (10) comprenant en outre :
un dispositif (62) d'entrée d'utilisateur configuré pour qu'un utilisateur saisisse une sélection pour qu'un espacement longitudinal entre les trous soit non uniforme tout en maintenant uniforme l'espacement latéral à l'intérieur de chaque ligne.

3. Dispositif médical (10) selon la revendication 1, comportant en outre :
un capteur (54) de mouvement configuré pour mesurer un paramètre de mouvement, comportant une vitesse longitudinale ou un déplacement longitudinal, du boîtier (36) par rapport à la surface (11) ; et
une commande (60) configurée pour commander le taux de répétition, d'après le paramètre de mouvement mesuré, de telle façon que les trous d'ablation soient espacés suivant la direction longitudinale.

4. Dispositif médical (10) selon la revendication 1, les impulsions électromagnétiques étant des impulsions laser présentant une longueur d'onde située dans la plage de 1,9 à 3,0 micromètres.

5. Dispositif médical (10) selon la revendication 3, le paramètre de mouvement comportant une vitesse longitudinale, et le capteur (54) étant configuré pour délivrer un signal de vitesse indicatif de la vitesse longitudinale, et la commande (60) étant configurée pour commander le taux de répétition en fonction de la vitesse longitudinale indiquée par le signal de vitesse, ou le paramètre de mouvement comportant un déplacement longitudinal, et le capteur (54) étant configuré pour délivrer un signal de déplacement indicatif du déplacement longitudinal, et la commande (60) étant configurée pour commander l'émission des impulsions en fonction du signal de déplacement.

6. Dispositif médical (10) selon la revendication 3, comportant en outre un galet (51 ; 52) qui est fixé de façon pivotante au boîtier (36) et configuré pour rouler contre la surface (11) tandis que le boîtier (36) est déplacé longitudinalement le long de la surface (11), de telle façon que la fréquence de rotation du galet (51; 52) soit proportionnelle à la vitesse longitudinale, et le capteur (54) de mouvement étant configuré pour mesurer un paramètre de mouvement angulaire, comportant une vitesse angulaire ou un déplacement angulaire, du galet (51 ; 52).

7. Dispositif médical (10) selon la revendication 6, le paramètre de mouvement angulaire étant la vitesse angulaire du galet (51 ; 52), et la commande (60) étant configurée pour commander le taux de répétition en fonction de la vitesse angulaire, ou le paramètre de mouvement angulaire étant le déplacement angulaire du galet (51 ; 51), et la commande (60) étant configurée pour commander l'émetteur (40) de façon à espacer les impulsions en fonction du déplacement angulaire.

8. Dispositif médical (10) selon la revendication 6, le galet étant un premier galet (51) sur un premier côté du boîtier (36), le paramètre de mouvement angulaire étant un premier paramètre de mouvement angulaire, le capteur (54) de mouvement comprenant un deuxième galet (52) sur un deuxième côté latéralement opposé du boîtier (36), le deuxième galet (52) étant fixé de façon pivotante au boîtier (36) pour tourner indépendamment de la rotation du premier galet (51), et le deuxième galet (52) étant configuré pour rouler contre la surface (11), et le capteur (54) de mouvement étant configuré pour mesurer un deuxième paramètre de mouvement angulaire, comportant la vitesse angulaire ou le déplacement angulaire, du deuxième galet (52).

9. Dispositif médical (10) selon la revendication 8, la commande (60) étant configurée pour commander le taux de répétition en fonction à la fois du premier paramètre de mouvement et du deuxième paramètre de mouvement angulaire.

10. Dispositif médical (10) selon la revendication 8, les impulsions émises par l'émetteur (40) générant des lignes (12) s'étendant latéralement de trous d'ablation, et la commande (60) étant configurée pour commander le nombre de trous d'ablation dans chaque ligne en fonction à la fois du premier paramètre de mouvement angulaire et du deuxième paramètre de mouvement angulaire.

11. Dispositif médical (10) selon la revendication 6, la commande (60) étant configurée pour faire chuter le taux de répétition à zéro en réaction à une inversion de sens du galet.

12. Dispositif médical (10) selon la revendication 6, le galet (51 ; 52) étant transparent.

13. Dispositif médical (10) selon la revendication 3, les impulsions comprenant des impulsions qui sont espacées latéralement pour produire des lignes (12) s'étendant latéralement de trous d'ablation qui, lorsqu'elles sont combinées avec le mouvement longitudinal du boîtier, donnent une matrice des trous (13) alignés en lignes (12) s'étendant latéralement et en colonnes s'étendant longitudinalement.

14. Dispositif médical (10) selon la revendication 13, l'émetteur (40) d'impulsions étant configuré pour générer les trous de chaque ligne simultanément ou séquentiellement.

15. Dispositif médical (10) selon la revendication 13, l'émetteur (40) comprenant un élément optique diffractif (41) configuré pour séparer l'énergie électromagnétique en une ligne s'étendant latéralement d'impulsions électromagnétiques simultanées qui génèrent simultanément les trous.

16. Dispositif médical (10) selon la revendication 13, la commande (60) étant configurée pour que les trous d'ablation soient espacés uniformément suivant la direction longitudinale ou soient espacés non uniformément suivant la direction longitudinale.

17. Dispositif médical (10) selon la revendication 13, la commande (60) étant configurée pour que les trous d'ablation soient espacés uniformément suivant la direction latérale ou soient espacés non uniformément suivant la direction latérale.
